# EUROPÄISCHE PATENTSCHRIFT

(11) **EP 0 980 229 B1**
(45) Veröffentlichungstag und Bekanntmachung des Hinweises auf die Patenterteilung: **05.11.2003**
(21) Anmeldenummer: 98922725.1
(22) Anmeldetag: 16.04.1998
(51) Int. Cl.: A61B 17/02, A61B 17/11

(54) **ANORDNUNG ZUM LOKALEN RUHIGSTELLEN EINES SCHLAGENDEN HERZENS**
DEVICE FOR LOCALLY IMMOBILIZING A BEATING HEART
SYSTEME D'IMMOBILISATION LOCALE D'UN COEUR EN TRAIN DE BATTRE

(30) Priorität: 29.04.1997 DE 29707567 U
(43) Veröffentlichungstag der Anmeldung: 23.02.2000
(73) Patentinhaber: Riess, Andreas G., 22850 Norderstedt (DE)
(72) Erfinder: Riess, Andreas G., 22850 Norderstedt (DE)
(74) Vertreter: Böhme, Ulrich, Dr. Dipl.-Phys.
(86) Internationale Anmeldenummer: EP9802235
(87) Internationale Veröffentlichungsnummer: WO98048703

(56) Entgegenhaltungen:
- EP-A- 0 820 721
- EP-A- 0 944 356
- WO-A-95/17127
- WO-A-97/10753

## Beschreibung

Die Erfindung betrifft eine Anordnung zum lokalen Ruhigstellen eines schlagenden Herzens, insbesondere zum Zwecke der Herstellung einer Anastomose zwischen einem Bypassconduit und einem Koronargefäß der Vorderwand der linken Herzkammer, mit einer gabelartigen Plattform mit zwei im wesentlichen parallel verlaufenden Gabelblättern, welche zwischen sich einen Zwischenraum ausbilden.

Minimal invasive Operationstechniken liegen im Trend und erfahren gerade heute vor dem Hintergrund der Notwendigkeit zur Kosteneinsparung reges Interesse. Dabei geht es jedoch nicht ausschließlich um die Kosten. Auch die Verringerung des prä-, intra- und postoperativen Traumas, die verkürzten Operations- und Narkosezeiten, die schnellere Wundheilung, kürzere Liegezeiten und weniger Wundschmerz sowie das kosmetische Ergebnis sind wichtige Argumente für minimal invasive Operationstechniken. In allen Bereichen der Chirurgie gibt es Bestrebungen, die Standardprozeduren durch minimal invasive Varianten zu ersetzen. Beispielsweise sei nur auf die videokontrollierte Trokar-Technik für gynäkologische Eingriffe verwiesen.

Herzchirurgische Operationen mit ihrer gemessen an der Größe des Eingriffes geringen Mortalität und Morbidität wurden erst durch die Entwicklung der Herz-Lungen-Maschine in den 50er Jahren möglich. Diese erlaubt für einen Zeitraum von mehreren Stunden Operationen an einem ruhig gestellten und blutleeren Herzen vorzunehmen. Für mehrere Jahrzehnte waren Operationen am Herzen mit Herz-Lungen-Maschine der Goldstandard.

In einer wesentlichen Fortentwicklung wurden später bestimmte Koronaroperationen in minimal invasiven Operationstechniken am schlagenden Herzen vorgenommen,
wobei der Einsatz der Herz-Lungen-Maschine überflüssig wurde. Die Motivation für dieses Vorgehen war das zunehmende Wissen um die Nebenwirkungen und Nachteile des kardiopulmonalen Bypass (CPB). Der Kontakt des Blutes mit Kunststoffoberflächen der Herz-Lungen-Maschine führt zu einer Aktivierung der sogenannten Gerinnungskaskade und des Komplementsystems. Um zu verhindern, daß es dadurch bedingt zu einer Blutgerinnselbildung in der Herz-Lungen-Maschine und dadurch zum Verschluß derselben kommt, sind hohe Dosen an Heparin notwendig. Durch die dadurch bewirkte komplette Aufhebung der Blutgerinnung kann es während und nach Eingriffen mit Herz-Lungen-Maschinen zu Blutungskomplikationen kommen. Dadurch kann die Verabreichung von Fremdblut mit all ihren möglichen Konsequenzen (Hepatitis, HIV, u.a.) notwendig werden. Auch die Blutplättchen, welche für eine normale Blutgerinnung essentiell sind, werden durch einen Eingriff mit Herz-Lungen-Maschine zum Teil erheblich in ihrer Anzahl und auch in ihrer Funktion beeinträchtigt, welches wiederum das Risiko für Blutungskomplikationen ansteigen läßt.

So verwundert es nicht, daß bei randomisierten prospektiven Studien mit größeren Anzahlen von Patienten ein Ergebnis war, daß die Patienten, welche am schlagenden Herzen ohne Herz-Lungen-Maschine operiert worden waren, postoperativ einen statistisch signifikant geringeren Blutverlust hatten, als die Patienten, welche mit Herz-Lungen-Maschine operiert wurden. Die Tatsache, daß bei Eingriffen mit Herz-Lungen-Maschine in der Regel das gesamte Brustbein eröffnet werden muß, kann zu postoperativen Schmerzen im Wundgebiet, aber auch zu Wundheilungsstörungen und Instabilitäten des Brustbeines führen. Weitere Nebenwirkungen von Eingriffen mit Herz-Lungen-Maschine sind neurologische Komplikationen, die in Zusammenhang mit der extrakorporalen Zirkulation gebracht werden. So können kleine Mikrogerinnsel, aber auch Luftembolien in Hirnarterien gelangen und dort Schlaganfälle auslösen. Eine weitere Quelle von thromboembolischen Komplikationen können feine Verkalkungen im Bereiche der Hauptschlagader sein, die sich durch die Manipulationen an derselben (Anschluß an die Herz-Lungen-Maschine und Abklemmung bzw. seitliche Ausklemmung der Hauptschlagader) abgesprengt werden können. Außerdem ist es bekannt, daß nicht wenige Patienten nach einem Eingriff mit Herz-Lungen-Maschine und kardioplegischem Herzstillstand geringgradige neurologische Ausfälle bzw. psychiatrische Auffälligkeiten (bis zu 30 Prozent) aufweisen können.

Dem gegenüber bietet die minimal invasive Versorgung des wichtigsten Gefäßes der linksventrikulären Vorderwand (LAD) ohne Herz-Lungen-Maschine zahlreiche Vorteile. Die Operation ist bei einem geübten Chirurgen schneller durchzuführen, als ein Eingriff mit Herz-Lungen-Maschine. Die Patienten haben eine kleinere Narbe und somit wird ein kosmetisch besseres Ergebnis erzielt. Das Brustbein behält einen Teil seiner Stabilität, da es nur partiell eröffnet wird. Dies bedingt weniger Wundschmerzen und ermöglicht eine in der Regel unkomplizierte Verheilungsphase des Knochens. Bei der LIMA/LAD (Brustbeinschlagader/Koronargefäß) Prozedur wird das wichtigste Gefäß des Herzens (LAD) mit dem besten Bypassconduit (LIMA) versorgt. Bis zu 80 Prozent des gesamten Blutbedarfs des Herzens kann durch den LAD gedeckt werden. Die meisten Patienten sind nach einer Single-LIMA auf den LAD, auch wenn weitere Stenosen in kleineren Ästen bestehen, nach erfolgter Operation beschwerdefrei, auch wenn die anderen Stenosen unbehandelt bleiben. Dennoch sollten diese Stenosen, sofern sie denn vorhanden sind, nach erfolgter minimal invasiver LIMA/LAD Prozedur aus prognostischer Sicht mit einem dann geringeren Risiko dilatiert worden, da zuvor der LAD versorgt worden ist. Betrachtet man die Offenheitsraten der verschiedenen Bypasstypen auf die verschiedenen Herzgefäße, so wird das Obengesagte nochmals verdeutlicht. Die Brustbeinschlagaderversorgung des LAD hat eine 10-Jahres-Offenheitsrate von über 93 Prozent. Dem gegenüber können die Venenbypässe schon nach wenigen Jahren Veränderungen der Gefäßinnenwände zeigen, und die Offenheitsrate von Venenbypässen liegt, je nachdem auf welches Gefäß sie genäht wurden, nur zwischen 40 bis 80 Prozent für 10 Jahre.

Weitere Vorteile bei der minimal invasiven Chirurgie sind die kurzen Narkosezeiten, eine in der Regel auf dem Operationstisch erfolgende Extubation, der nur wenige Stunden dauernde Aufenthalt auf der Intensivstation und ein Gesamthospitalaufenthalt von ca. zwei bis vier Tagen. Dies ist für den Patienten vorteilhaft und Kosten können reduziert werden. Ferner kommt es bedingt durch das kleinere Wundgebiet zu geringeren Verwachsungen zwischen Herzbeutel und Herz, was für eventuelle spätere Re-Operationen von Wichtigkeit sein kann. Außerdem wird in Studien berichtet, daß das Auftreten von Herzrhythmusstörungen im postoperativen Zeitraum nach minimal invasiven herzchirugischen Eingriffen geringer ist.

Zur Herstellung der Anastomose zwischen LIMA und LAD muß am schlagenden Herz der Anastomosenbereich ruhiggestellt werden, um die ca. 15 Stiche in einem Bereich von wenigen Millimetern mit der erforderlichen Präzision vornehmen zu können.

Die LIMA/LAD Prozedur am schlagenden Herzen ist bekannt und wird praktiziert. Hierbei wird am beatmeten Patienten eine sogenannte Mini-Sternotomie vorgenommen. Es wird eine ca. acht Zentimeter lange Hautinzision beginnend ca. zwei Zentimeter oberhalb des Schwertfortsatzes bis auf Höhe des vierten Interkostalraumes (ICR) durchgeführt. Anschließend wird eine partielle mediane Sternotomie bis in den linken dritten ICR vorgenommen. Die LIMA wird unter direkter Sicht des Auges bis ca. zum zweiten ICR präpariert. Anschließend wird die Gerinnungszeit des Blutes verlängert, indem 5000 bis 7500 Einheiten Heparin intravenös verabreicht werden. Anschließend wird distal und proximal des für die Anastomose gewählten Bereiches der LAD angeschlungen und dadurch occludiert. Bei der anschließend durchzuführenden End-zu-Seit-Anastomose zwischen LIMA und LAD mit einer fortlaufenden 8-0 Naht ist es für die Qualität der Anastomose und damit für den Erfolg der Operation insgesamt von größter Bedeutung, wie gut es gelingt den Anastomosenbereich zu stabilisieren.

Es wurden dafür verschiedene Werkzeuge entwickelt, die zum Teil von einem Assistenten zu halten oder auf verschiedene Weise festgesetzt sind. Diese Werkzeuge sind entweder unter hohem Druck auf den Operationsbereich aufzupressen oder besitzen Saugnäpfe, mit deren Hilfe das Herz angehoben wird (WO 97/10753). In beiden Fällen ist es jedoch besonders nachteilig, daß es durch die starken Druck- oder Saugkräfte zu Einrissen oder Hämatomen am Herzgewebe kommen kann.

EP-A-944 356, ein Dokument, das unter Art. 54(3) EPÜ fällt, offenbart eine Ausführungsform mit Gabelblättern, die Öffnungen aufweisen und eine andere Ausführungsform mit gabelartig angeordneten Armen, die eine Befestigungsvorrichtung für einen Faden aufweist.

Es ist Aufgabe der vorliegenden Erfindung eine verbesserte Anordnung der o.g. Art zur Verfügung zu stellen, welche die obengenannten Nachteile beseitigt.

Diese Aufgabe wird durch eine Anordnung der o.g. Art mit den in Anspruch 1 gekennzeichneten Merkmalen gelöst.

Dazu ist es bei einer Anordnung der oben genannten Art erfindungsgemäß vorgesehen, daß der Zwischenraum eine Breite aufweist, die der 1- bis 5-fachen Breite eines in dem Zwischenraum anzuordnenden Koronargefäßes entspricht, wobei angrenzend zum Zwischenraum auf den Gabelblättern wenigstens je eine Öffnung vorgesehen ist, wobei ferner Mittel zum Umschlingen des Koronargefäßes vorgesehen sind, welche durch die wenigstens zwei Öffnungen hindurchführbar und an wenigstens einer auf den Gabelblättern vorgesehenen Befestigungsvorrichtung befestigbar sind. Die Idee ist dabei im wesentlichen, auf das Niederdrücken zum Zweck der Stabilisierung ganz zu verzichten. Vielmehr wird die Plattform auf den LAD gesenkt und dieser zum Zweck der Arretierung im Bereich der kreisartigen Erweiterung angezügelt. Das Konzept zielt darauf, die Friktion so gering wie möglich zu halten und dadurch das Herz so wenig wie möglich in seiner Bewegung zu behindern. Dabei ist die Fläche der Gabelblätter vergleichsweise groß und auf der Unterseite eben und glatt. Der Kernpunkt ist, daß die Ruhigstellung des LAD einzig durch die Anzügelung, und damit das Hereinziehen des Anastomosenbereiches in die kreisartige Erweiterung des Plattformspaltes bewerkstelligt wird. Friktionskräfte sind für diese Form der Fixierung nicht erforderlich.

Dies hat den Vorteil, daß bei verbesserter Stabilisierung und für den Operateur optimaler Zugänglichkeit des zu operierenden Koronargefäßes gleichzeitig die Traumatisierung des Herzgewebes minimiert wird.

Weitere Vorteile der erfindungsgemäßen Anordnung liegen in folgendem:

Es wird eine Mini-Sternotomie mit einer Länge von beispielsweise 8 bis 10 cm ermöglicht. Dies vermeidet die asymmetrische Öffnung unter Durchtrennung der Muskulatur sowie des Gefäß/Nervenbündels des ICR. Dadurch werden die zum Teil erheblichen Wundschmerzen einer lateralen Thorakotomie vermieden, die oft die Verabreichung von Opiaten über einen längeren Zeitraum nötig machen. Im Vergleich dazu treten bei der medianen Sternotomie und insbesondere bei der Mini-Sternotomie erstaunlich geringfügige postoperative Schmerzen auf, so daß der postoperative Analgetikabedarf gering ist.

Es erfolgt eine maximale Ruhigstellung des Anastomosenbereiches bei gleichzeitig optimaler Justierbarkeit, minimaler Beeinträchtigung der Herzfunktion durch die Anordnung und minimaler Traumatisierung des Herzens durch den Kontakt mit der erfindungsgemäßen Anordnung. Der Operateur hat eine freie Sicht auf das Operationsfeld und die Herzbewegung ist visuell ausgeblendet. In der operativen Praxis hat es sich gezeigt, daß die Aktion des schlagenden Herzens eine visuelle Beeinträchtigung des Operationsfeldes darstellt, welche die Konzentration auf den vergleichsweise kleinen Anastomosenbereich erschwert.

Die Möglichkeit der Fixierung des LAD an den Gabelplatten mit entsprechenden Mitteln bringt eine erheblich höhere Anwendungssicherheit und eine verbesserte Ruhigstellung des Operationsbereiches ohne die Notwendigkeit von Anpreßdruck auf die Herzmuskulatur.

Die Anordnung ist schnell und einfach montierbar und sekundenschnell demontierbar. Wenn es bei der Occlusion des LAD zu stärkeren hämodynamischen Beeinträchtigungen der Pumpfunktion des Herzens bzw. zu malignen Herzrhythmusstörungen kommt, kann es erforderlich werden, die Operationsstrategie zu wechseln und die mediane Sternotomie zu komplettieren und mit Hilfe der Herz-Lungen-Maschine die Operation fortzusetzen. Um den Zeitraum einer hämodynamischen Beeinträchtigung und damit die Phase einer Sauerstoffmangelsituation des Gehirns möglichst kurz zu halten ist es erforderlich, daß eine eingebrachte Stabilisierungsplattform sekundenschnell demontiert werden kann.

Durch die vergleichsweise geringe Auflagefläche bisheriger Lösungen kommt es während des Drückens auf den Herzmuskel an den umlaufenden Rändern zu mehr oder weniger starken Knickungen und damit zur Traumatisierung des Herzmuskels. Erfindungsgemäß ist die Fläche der Gabelblätter so groß bemessen, daß es nicht zum Knikken des Herzmuskels an den umlaufenden Rändern kommen kann. Vielmehr wird der LAD mit seinem Begleitbewebe nur im Bereich des Spalts vom Rand der Gabelblätter gehalten. Die kombinierte Fläche der beiden Plattformblätter beträgt beispielsweise 7 bis 30 Quadratzentimeter, bei einer bevorzugten Ausführungsform 15 Quadratzentimeter.

Vorzugsweise Weitergestaltungen des Verfahrens sind in den Ansprüchen 2 bis 16 beschrieben.

Zweckmäßigerweise ist wenigstens eine kreisartige Erweiterung des Zwischenraumes vorgesehen. Diese erlaubt dem Operateur einen verbesserten Zugang zum in der Anordnung fixierten Koronargefäß. Die kreisartige Erweiterung hat vorzugsweise einen Durchmesser von 8 mm bis 12 mm, insbesondere von 10 mm.

In besonders vorteilhafter Weise ist das Befestigungsmittel wenigstens ein Profilknopf mit pilzartigem Querschnitt. Dies erlaubt ein einfaches und schnelles Belegen der Mittel zum Umschlingen des Koronargefäßes an der erfindungsgemäßen Anordnung.

Dadurch, daß die wenigstens zwei Öffnungen je auf einem Gabelblatt ausgebildet sind, kann ein zu operierenden Gefäß mit dem Mittel zum Umschlingen das Gefäß im Zwischenraum einspannen und somit wirksam stabilisieren bzw. ruhigstellen.

Zeckmäßigerweise sind die wenigstens zwei Öffnungen Bohrungen in den Gabelblättern.

Ein Ein- und Ausfädeln der Mittel zum Umschlingen des Koronargefäßes durch die Öffnungen in den Gabelblättern wird dadurch vermieden, daß in den Gabelblättern Schlitze vorgesehen sind, welche ausgehend vom Zwischenraum zu je einer Öffnung verlaufen. Zweckmäßigerweise sind dabei die Schlitze jeweils haken- oder S-förmig ausgebildet, so daß ein unbeabsichtigtes Herausrutschen der Mittel zum Umschlingen des Koronargefäßes aus den Öffnungen verhindert ist.

Eine besonders schonende und sichere Auflage der Anordnung am Herzen wird dadurch erzielt, daß wenigstens ein Gabelblatt an einer am Herzen aufliegenden Seite zum Zwischenraum hin angefast ist. Der Anfaswinkel beträgt vorzugsweise 5 bis 15 Grad, insbesondere 10 Grad.

Zweckmäßigerweise weist der Zwischenraum eine Breite von 4 mm bis 10 mm, insbesondere von 6,5 mm, auf. Hierdurch bietet der Zwischenraum in besonders vorteilhafter Weise nur Platz für eine Gewebefalte und somit kann ein Koronargefäß positionsgenau in dem Zwischenraum ohne große aufzuspannende Längen für das Mittel zum Umschlingen des Koronargefäßes fixiert werden.

Zweckmäßigerweise ist das Mittel zum Umschlingen des Koronargefäßes wenigstens ein Faden oder wenigstens ein Vesselloop (hohler Gummizügel).

In einer besonders bevorzugten Weiterbildung der Erfindung weist der Zwischenraum eine Breite auf, die der 1- bis 2-fachen, 1- bis 3-fachen oder 2- bis 3-fachen Breite des in dem Zwischenraum anzuordnenden Koronargefäßes entspricht.

Zweckmäßigerweise weist der Zwischenraum eine derartige Breite auf, daß in diesem ein Koronargefäß derart anordbar ist, daß dieses seitliche Abstände zu den Gabelblättern aufweist, die jeweils gleich oder kleiner als die Koronargefäßbreite sind.

Die Anordnung fügt sich dadurch besonders gut an die Oberfläche des Herzens an, daß die Gabelblätter über eine Brücke miteinander verbunden sind, welche zwischen zwei Schenkeln, an denen je ein Gabelblatt angeordnet ist, eine im wesentlichen halbkreisförmige Ausnehmung aufweist. Diese weist bevorzugt einen Radius von 2 mm bis 5 mm (halbe Spaltbreite), insbesondere von 3,25 mm auf.

Nachstehend wird die Erfindung anhand der beigefügten Zeichnungen näher erläutert. Diese zeigen in
- Fig. 1: eine erfindungsgemäße Anordnung in perspektivischer Ansicht,
- Fig. 2: in Vorderansicht,
- Fig. 3: in Aufsicht,
- Fig. 4: eine Befestigungsvorrichtung in Schnittansicht,
- Fig. 5: eine erfindungsgemäße Anordnung mit Feststellmitteln in perspektivischer Ansicht,
- Fig. 6: in einer weiteren perspektivischen Ansicht,
- Fig. 7: ein Auslegergelenk in Explosionsdarstellung,
- Fig. 8: eine während einer Operation montierte Anordnung in perspektivischer Ansicht,
- Fig. 9 und 10: eine Illustration der Fixierung eines Koronargefäßes an einer erfindungsgemäßen Anordnung in perspektivischer Ansicht,
- Fig. 11: eine vergrößerte Darstellung eines in der erfindungsgemäßen Anordnung fixierten Gefäßes,
- Fig. 12A bis 12D: eine Schnittansicht verschiedener Herzzustände bei in der erfindungsgemäßen Anordnung fixiertem Koronargefäß,
- Fig. 13: eine zweite Ausführungsform einer erfindungsgemäßen Anordnung,
- Fig. 14: eine dritte Ausführungsform einer erfindungsgemäßen Anordnung und
- Fig. 15 und 16: bevorzugte Ausführungsformen von Gabelflächen.

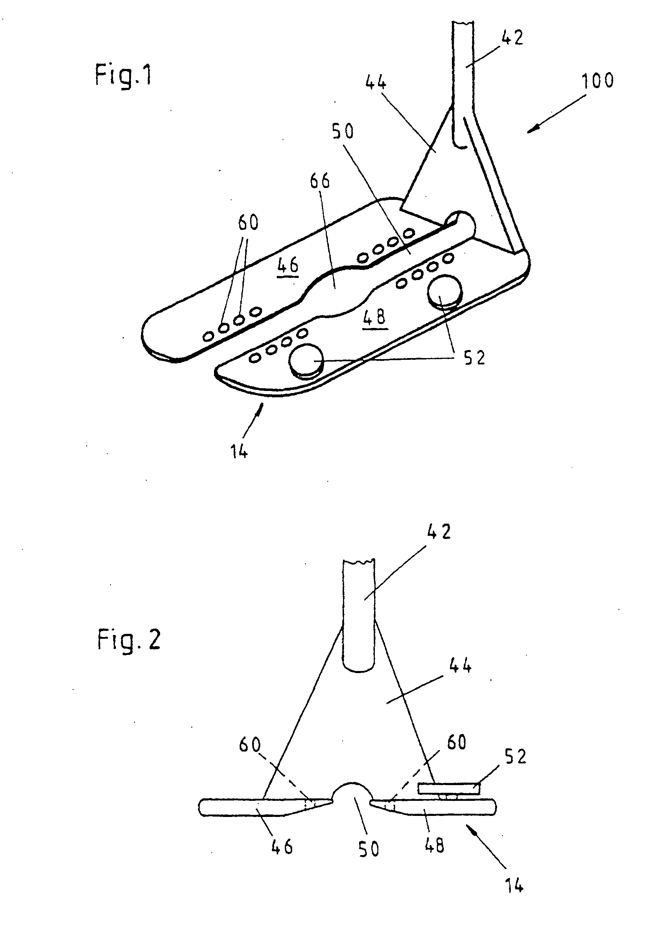

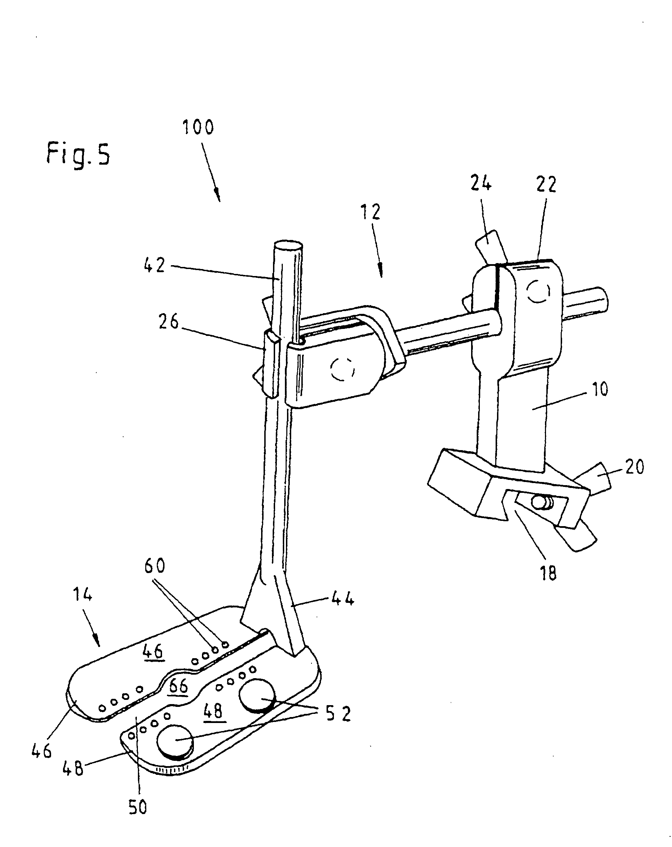

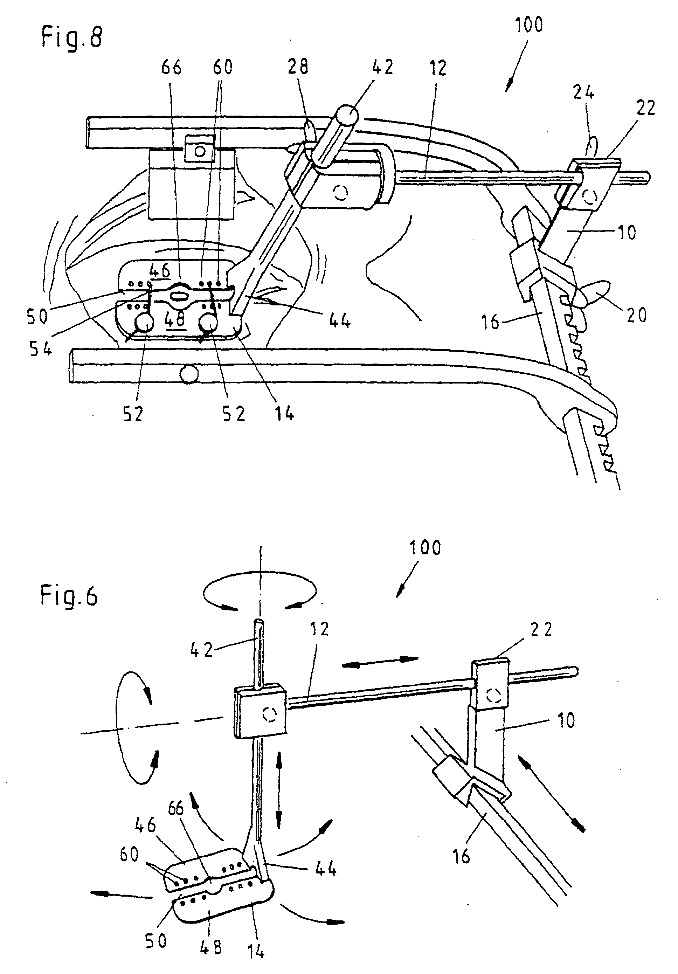

Die in Fig. 1 bis 3 und 5 bis 8 dargestellte erfindungsgemäße Anordnung 100 umfaßt im wesentlichen drei Teile: Eine Säule 10, einen Ausleger 12 und eine Plattform 14. Die Säule 10 gleitet mit einem Anschluß 16, z. B. auf der Basis eines BIRNBAUM-Sperrers 16 und läßt sich durch Anziehen einer Flügelschraube 20 in jeder erreichbaren Position festsetzen. Im 90 Grad Winkel trägt sie den Ausleger 12 in einem Gelenk 22, welches es erlaubt, den Ausleger 12 vor- und zurückzuschieben, sowie ihn um seine Längsachse um jeden beliebigen Winkel zu drehen (vgl. Fig. 6). Das Gelenk 22 läßt sich durch Anziehen einer Flügelmutter 24 festsetzen.

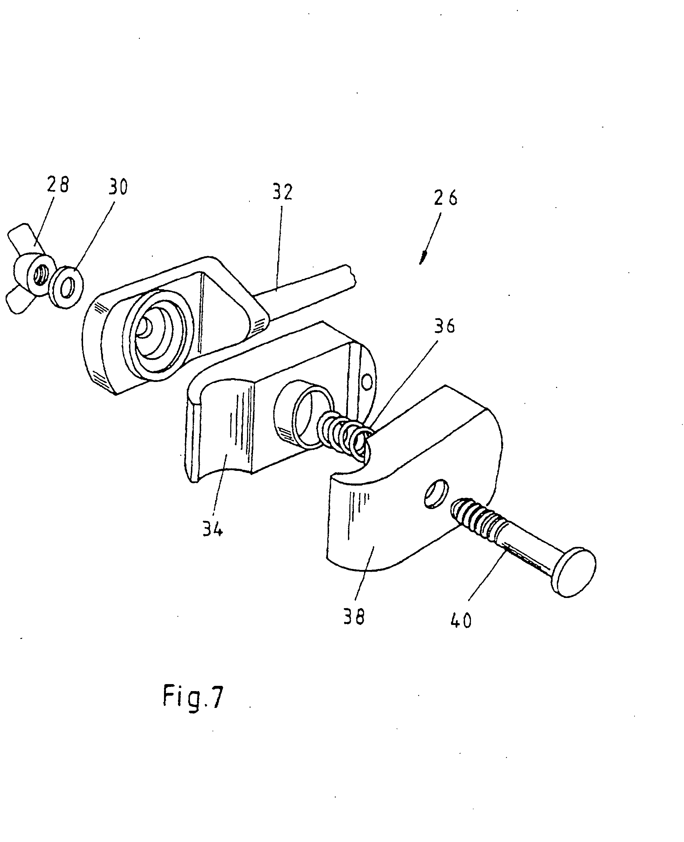

Am Ausleger 12 ist an einem Ende ein weiteres Gelenk 26 angeordnet. Dieses ist in Fig. 7 im Detail dargestellt und umfaßt eine Flügelmutter 28, eine Unterlegscheibe 30, einen Auslegerstiel 32, eine innere Schale 34, eine Druckfeder 36, eine äußere Schale 38 und einen Schraubenbolzen 40, beispielsweise einen M6-Bolzen.

Die Freiheitsgrade von Säule 10 und Stiel 12 bzw. 32 erlauben es, dieses Gelenk 26 auf einer festen Ebene über dem Operationsfeld beliebig zu positionieren und in einem beliebigen Raumwinkel auszurichten (vgl. Fig. 6). Weiter ist dieses Gelenk 26 so beschaffen, daß es einen Stiel 42 der Plattform 14 aufnehmen kann und dieser in Sekundenschnelle durch Anziehen der Flügelmutter 28 festsetzbar ist.

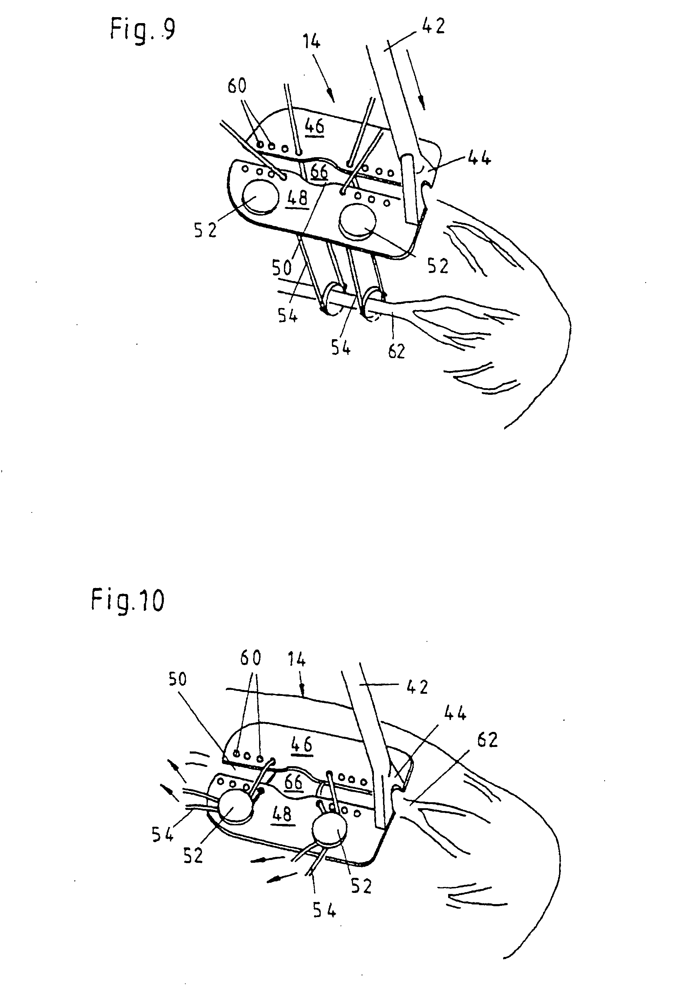

Die Plattform 14 umfaßt den vorgenannten Stiel 42, der an seinem unteren Ende eine Brücke 44 trägt, deren Basen wiederum zwei Plattformblätter 46 und 48 tragen. Die Plattform 14 bildet zwischen seinen Gabelblättern 46 und 48 einen Zwischenraum 50 aus, der zur Aufnahme eines Koronargefäßes dient, wie dies in den Fig. 9 bis 11 dargestellt ist. Der Zwischenraum hat beispielsweise eine Breite von 6,5 mm. Ferner sind auf wenigstens einem Gabelblatt 48 Befestigungsmittel 52 ausgebildet, die zur Aufnahme und zum Belegen von Umschlingungsmitteln 54 dienen (vgl. Fig. 10).

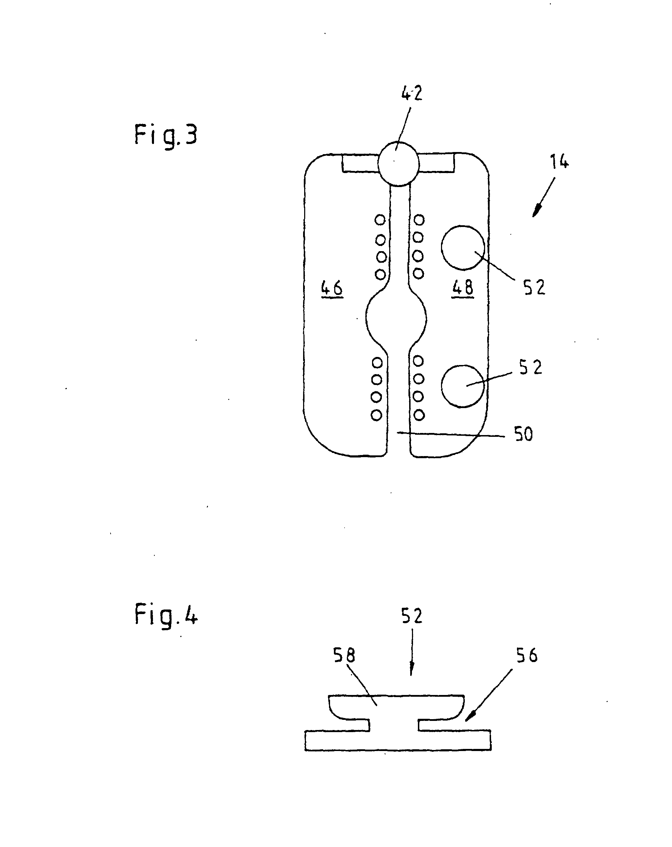

Die Befestigungsmittel sind beispielsweise pilzartig ausgebildet, wie in Fig. 4 dargestellt, wobei ein Spalt 56 unter dem Pilzkopf 58 zur Aufnahme und zum Befestigen der Umschlingungsmittel 54 dient. Dies ist beispielsweise aus den Fig. 8 und 10 ersichtlich.

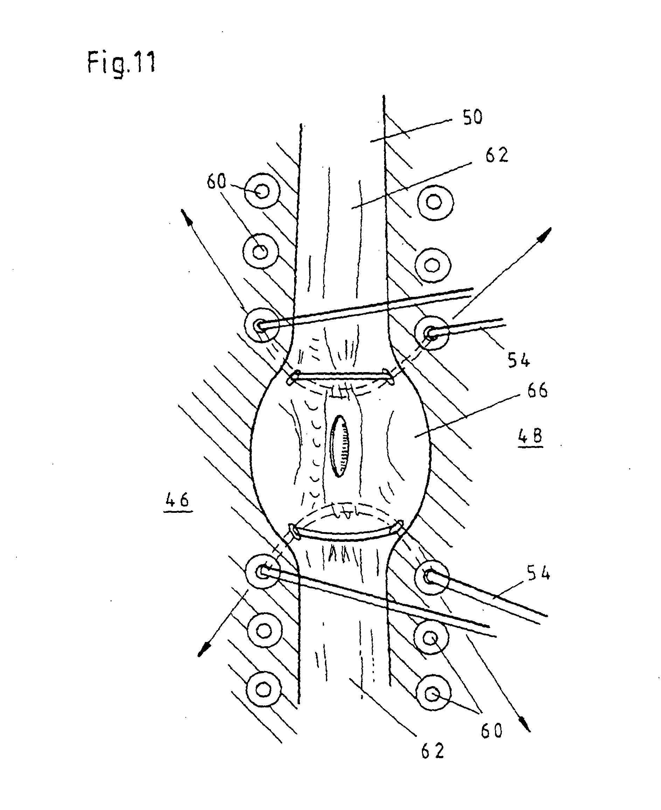

Auf den Gabel- bzw. Plattformblättern 46 und 48 sind ferner Öffnungen 60 ausgebildet, durch die die Umschlingungsmittel 54 durchführbar sind, wie in Fig. 9 bis 11 dargestellt. Die Öffnungen 60 dienen dabei als Lagerpunkte für die Mittel 54, die beispielsweise Vesselloops 54 sind, so daß das umschlungene Koronargefäß 62, wie aus den Fig. 9 bis 11 ersichtlich, im Spalt bzw. Zwischenraum 50 eingespannt und fixiert werden kann. Für eine gute Zugänglichkeit des Koronargefäßes 62 im Zwischenraum 50 weist letzterer ferner eine kreisartige Aufweitung 66 auf (vgl. insbesondere Fig. 11).

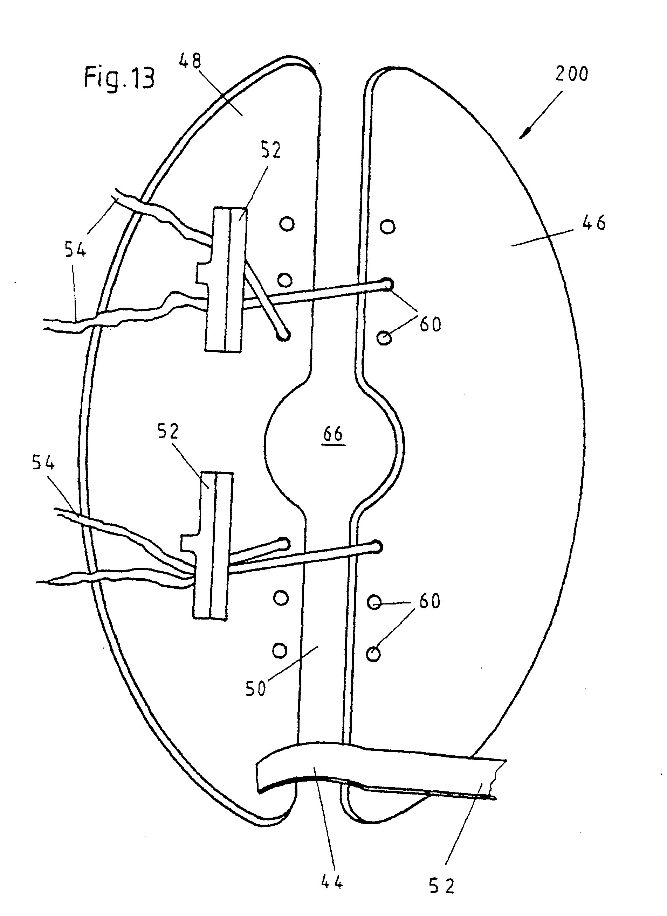

Fig. 13 zeigt eine zweite vorteilhafte Ausführung 200 der Plattform, wobei die Befestigungsmittel 52 stegförmig ausgebildet sind.

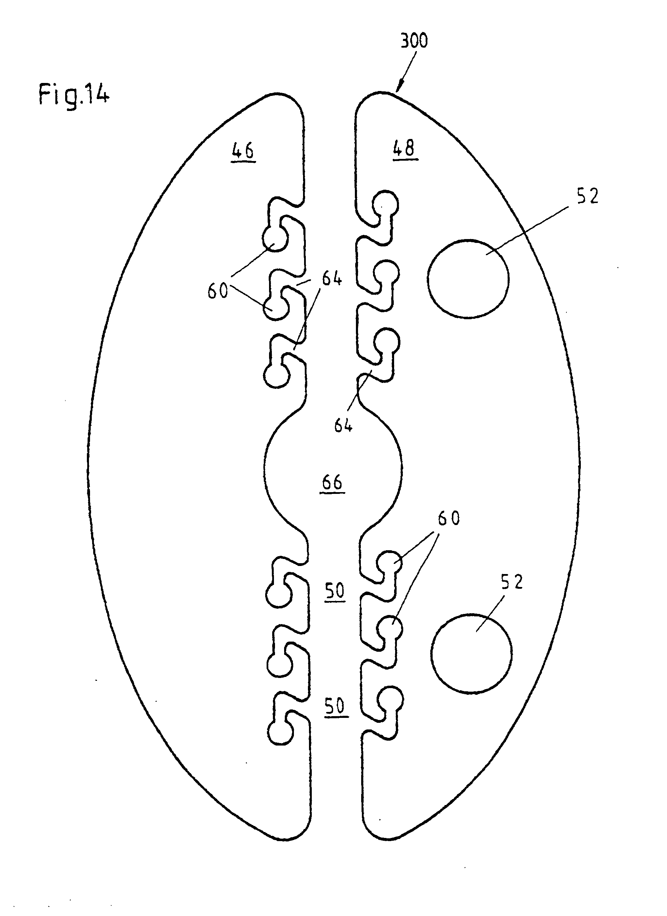

Fig. 14 zeigt eine dritte bevorzugte Ausführungsform der Plattform, wobei die Öffnungen 60 zusätzlich durch Schlitze 64 mit dem Zwischenraum 50 verbunden sind. Auf diese Weise kann ein mühsames Einfädeln der Vesselloops 54 in die Öffnungen 60 entfallen. Die Vesselloops 54 werden statt dessen Über die Schlitze 64 in den entsprechenden Öffnungen 60 plaziert. Hierbei sind die Schlitze 64 bevorzugt hakenförmig oder S-förmig ausgebildet, so daß ein unbeabsichtigtes Herausrutschen der Vesselloops 54 aus den Öffnungen 60 wirksam verhindert ist.

Handhabung, Funktionsweise und weitere Einzelheiten der erfindungsgemäßen Anordnung werden nachfolgend unter Bezugnahme auf die Fig. 8 bis 12 näher erläutert.

Die Anordnung besteht bevorzugt aus chirurgischem Stahl und die Oberfläche ist durch Glasperlenstrahlen behandelt.

Die Säule 10 trägt den Ausleger 12 und stellt den Übergang zum jeweiligen Sperrer 16, 18, beispielsweise einen BIRNBAUM-Sperrer dar. Es ist bevorzugt, auswechselbare Adaptermodule oder einen Universaladapter vorzusehen, um die erfindungsgemäße Anordnung an alle im Gebrauch befindlichen Sperrer anschließen zu können.

In einer vorteilhaften Weiterbildung ist ein Kugelgelenk vorgesehen, das es ermöglicht, den Ausleger 12 in jeden sinnvollen Raumwinkel zu drehen, sowie ihn im Gelenk vor- und zurückschieben zu können. Eine solche Variation vereinfacht die Justierung der Plattform.

Die Konstruktion des Auslegergelenks 26 berücksichtigt bei seinen Abmessungen die Anforderungen an hohe Stabilität, freie Sicht, schnelle Aufnahme und Arretierung sowie ebenso schnelle Freigabe des Plattformstiels 42.

Die im Gelenk 26 enthaltene Druckfeder 36 wird vorzugsweise vollständig von einer Hülse umschlossen, um im Fall eines Federbruchs das unkontrollierte Hinabfallen von Bruchstücken in die Brusthöhle unmöglich zu machen.

Um den Reibungswiderstand des Herzens so gering wie möglich zu gestalten, ist die Unterseite der Plattformblätter 46 und 48 vorzugsweise poliert.

Der Spalt 50 mit seiner runden Erweiterung 66 hält mit seinen stumpfen Schneiden den LAD 62. Die Ösen 60 für die Vesselloops 54 befinden sich in vier Viereranordnungen rechts und links der runden Erweiterung 66 entlang der Spaltkanten. Von großer Wichtigkeit für die Sicherheit ist das vollständige Entschärfen und Polieren dieser Ösen 66, damit ein Durchtrennen der Vesselloops 54 auf jeden Fall vermieden wird.

Zwei Knöpfe 52 dienen zur Arretierung der Vesselloops 66. Hierzu werden die Hohlschläuche oder hohle Gummizügel 54 um die Basis der Knöpfe 52 geschlungen und sind bereits nach einer Umdrehung fest arretiert.

Im Operationsbetrieb wird die Anordnung 100 ohne Plattform 14 angereicht, nachdem der LAD 62 vor und nach dem Anastomosenbereich mit Vesselloops 54 umschlungen wurde. Der Ausleger 12 befindet sich in einer auf Erfahrung beruhenden mittleren Position, das Säulengelenk 22 ist geschlossen, das Auslegergelenk 26 geöffnet und parallel zur Säule 10 ausgerichtet. Die Flügelmuttern 20, 24, 28 weisen zum Assistenten.

Die Anordnung 100 wird auf die Basis des BIRNBAUM-Sperrers 16 aufgesetzt und in einer auf Erfahrung beruhenden mittleren Position durch Anziehen der Flügelschraube 20 an der Basis der Säule 10 befestigt.

Durch abwechselndes Lösen und Schließen der Gelenke 22, 26 wird die Anordnung-100 so ausgerichtet, daß die Hülse des Auslegergelenks 26 senkrecht auf den Anastomosenbereich weist, um ca. Drei cm nach rechts versetzt.

Nun wird die Plattform 14 angereicht und die Vesselloops 54 eingefädelt (Fig. 9).

Dann wird der Plattformstiel 42 in das geöffnete Auslegergelenk 26 eingelegt und das Gelenk 26 geschlossen, jedoch noch nicht festgesetzt.

Es folgt das gefühlvolle Anzügeln, Zentrieren und Absenken der Plattform 14 unter ständiger Abwägung der Faktoren "minimale Traumatisierung des LAD 62 durch die Vesselloops 54", "minimale Behinderung der Herzaktion", "optimale Ausrichtung der Plattform 14 (tangential zur Herzoberfläche, Anastomosenbereich zentriert, Plattformspalt 50 parallel zum LAD 62)" und schließlich "optimale Ruhigstellung des Anastomosenbereichs".

Wie in Fig. 10 dargestellt werden anschließend die Vesselloops 54 an den Knöpfen 52 festgesetzt. Nun kann der Operateur mit dem Eingriff beginnen, wobei er ein optimal ruhiggestelltes und fixiert ausgerichtetes Koronargefäß 62 vor sich hat.

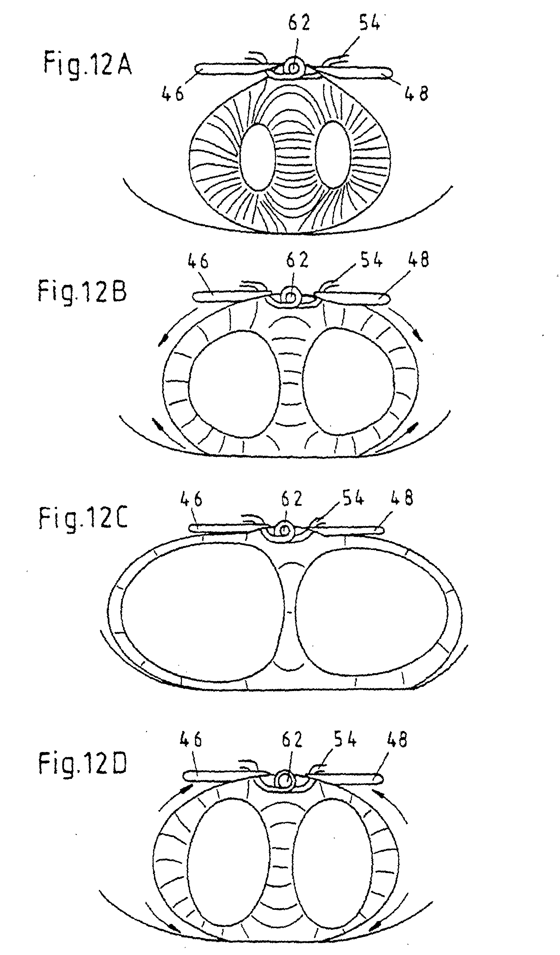

Fig. 12A bis 12D veranschaulichen die Situation bei fixiertem Koronargefäß 62 am schlagenden Herzen im kontraktierten Zustand (Fig. 12A), in der Expansionsphase (Fig. 12B), in expandiertem Zustand (Fig. 12C) und in der Kontraktionsphase (Fig. 12D). Hier wird deutlich, daß trotz unbehindert schlagendem Herzen das Koronargefäß 62 praktisch vollständig ruhiggestellt ist.

Fig. 9 zeigt im Detail das Anbringen der erfindungsgemäßen Anordnung am Herzen. Der LAD 62 wird rechts und links neben dem Anastomosenbereich von Vesselloops 54 umschlungen und diese in geeignete Ösen bzw. Öffnungen 60 der Plattform 14, 46, 48 gefädelt. Dann wird die Plattform abgesenkt, wobei die Vesselloops 54 leicht unter Spannung gehalten werden. Wenn die Plattform 14, 46, 48 aufgesetzt ist (vgl. Fig. 10), werden die Vesselloops 54 derart unter Spannung gesetzt, daß der LAD 62 verschlossen und der Anastomosenbereich in der Erweiterung 66 des Plattformspaltes 50 zentriert ist.

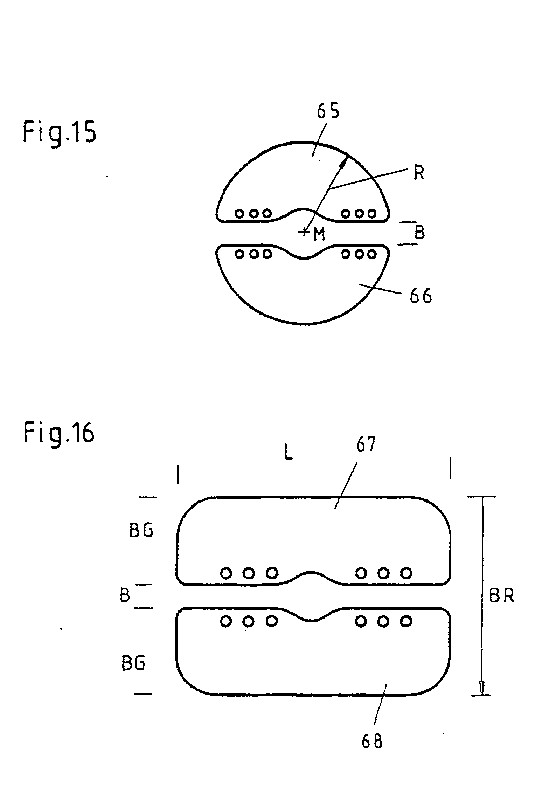

Fig. 15 und 16 zeigen schematisch zwei Ausführungsformen der Gabelflächen als Anlageflächen. Bei der Ausführungsform gem. Fig. 15 ergänzen sich die beiden Gabelflächen 65, 66 zusammen mit dem Zwischenraum 50 zu einer Kreisfläche mit einem Außenradius R, wobei die Breite B des Zwischenraumes 50 etwa einem Drittel des Radius R entspricht. Die so gebildete Auflagefläche beträgt bevorzugterweise 7 cm².

Bei der Ausführungsform gemäß Fig. 16 bilden die beiden Gabelflächen 67, 68 mit dem Zwischenraum 50 etwa ein Rechteck mit der Länge L und einer Breite B, die der Summe der Breiten BG der Gabelflächen 67, 68 und der Breite B des Zwischenraumes 50 entspricht. Die so gebildete Auflagefläche beträgt bevorzugterweise 30 cm².

## Patentansprüche

1. Anordnung zum lokalen Ruhigstellen eines schlagenden Herzens mit einer gabelartigen Plattform (14) mit zwei im wesentlichen parallel verlaufenden Gabelblättern (46, 48), welche zwischen sich einen Zwischenraum (50) ausbilden, wobei der Zwischenraum (50) eine Breite aufweist, die der 1- bis 5-fachen Breite eines in dem Zwischenraum (50) anzuordnenden Koronargefäßes (62) entspricht, und wobei angrenzend zum Zwischenraum (50) auf den Gabelblättern (46, 48) wenigstens je eine Öffnung (60) vorgesehen ist, wobei ferner Mittel (54) zum Umschlingen des Koronargefäßes (62) vorgesehen sind, welche durch die wenigstens zwei Öffnungen (60) hindurchführbar und an wenigstens einer auf den Gabelblättern (46, 48) vorgesehenen Befestigungsvorrichtung (52) befestigbar sind.

2. Anordnung nach Anspruch 1, **dadurch gekennzeichnet, daß** wenigstens eine kreisartige Erweiterung (66) des Zwischenraumes (50) vorgesehen ist.

3. Anordnung nach Anspruch 2, **dadurch gekennzeichnet, daß** die kreisartige Erweiterung (66) einen Durchmesser von 8 mm bis 12 mm, insbesondere von 10 mm hat.

4. Anordnung nach einem der vorhergehenden Ansprüche, **dadurch gekennzeichnet, daß** das Befestigungsmittel (52) wenigstens ein Profilknopf mit pilzartigem Querschnitt ist.

5. Anordnung nach einem der vorhergehenden Ansprüche, **dadurch gekennzeichnet, daß** wenigstens je zwei Öffnungen (60) auf einem Gabelblatt (46, 48) ausgebildet sind.

6. Anordnung nach einem der vorhergehenden Ansprüche, **dadurch gekennzeichnet, daß** die wenigstens zwei Öffnungen (60) Bohrungen in den Gabelblättern (46, 48) sind.

7. Anordnung nach einem der vorhergehenden Ansprüche, **dadurch gekennzeichnet, daß** in den Gabelblättern (46, 48) Schlitze (64) vorgesehen sind, welche ausgehend vom Zwischenraum (50) zu je einer Öffnung (60) verlaufen.

8. Anordnung nach Anspruch 7, **dadurch gekennzeichnet, daß** die Schlitze (64) jeweils haken- oder S-förmig ausgebildet sind.

9. Anordnung nach einem der vorhergehenden Ansprüche, **dadurch gekennzeichnet, daß** wenigstens ein Gabelblatt (46, 48) an seiner Unterseite zum Zwischenraum (50) hin angefast ist.

10. Anordnung nach Anspruch 9, **dadurch gekennzeichnet, daß** der Anfaswinkel 5 bis 15 Grad, insbesondere 10 Grad, beträgt.

11. Anordnung nach einem der vorhergehenden Ansprüche, **dadurch gekennzeichnet, daß** der Zwischenraum (50) eine Breite von 4 mm bis 10 mm, insbesondere von 6,5 mm, aufweist.

12. Anordnung nach einem der vorhergehenden Ansprüche, **dadurch gekennzeichnet, daß** das Mittel (54) zum Umschlingen des Koronargefäßes (62) wenigstens ein Faden oder wenigstens ein Vesselloop ist.

13. Anordnung nach einem der vorhergehenden Ansprüche, **dadurch gekennzeichnet, daß** der Zwischenraum (50) eine Breite aufweist, die der 1- bis 2-fachen, 1-bis 3-fachen oder 2- bis 3-fachen Breite des in dem Zwischenraum (50) anzuordnenden Koronargefäßes (62) entspricht.

14. Anordnung nach einem der vorhergehenden Ansprüche, **dadurch gekennzeichnet, daß** der Zwischenraum (50) eine derartige Breite aufweist, daß in diesem ein Koronargefäß (62) derart anordbar ist, daß dieses seitliche Abstände zu den Gabelblättern (46, 48) aufweist die jeweils gleich oder kleiner als die Koronargefäßbreite sind.

15. Anordnung nach einem der vorhergehenden Ansprüche, **dadurch gekennzeichnet, daß** die Gabelblätter (46, 48) über eine Brücke (44) miteinander verbunden sind, welche zwischen zwei Schenkeln, an denen je ein Gabelblatt (46, 48) angeordnet ist, eine im wesentlichen halbkreisförmige Ausnehmung aufweist.

16. Anordnung nach Anspruch 15, **dadurch gekennzeichnet, daß** die halbkreisförmige Ausnehmung einen Radius von 2 mm bis 5 mm, insbesondere von 3,25 mm aufweist.

17. Anordnung nach einem der vorhergehenden Ansprüche, **dadurch gekennzeichnet, daß** die Flächen der beiden Gabelblätter (65, 66) und des Zwischenraumes (50) sich im wesentlichen zu einem Kreisring ergänzen, wobei der Außenrand der Kreissegmente ein Abstand bzw. Radius R von einem fiktiven Kreismittelpunkt M aufweist, der etwa dem Dreifachen der Breite B des Zwischenraumes (50) entspricht.

18. Anordnung nach Anspruch 17, **dadurch gekennzeichnet, daß** die Fläche der beiden Gabelblätter (65, 66) etwa 7 cm² beträgt.

19. Anordnung nach einem der Ansprüche 1 bis 16, **dadurch gekennzeichnet, daß** die Fläche der beiden Gabelblätter (67, 68) und des Zwischenraumes (50) sich im wesentlichen zu einem Rechteck ergänzen, wobei die Länge L der Fläche eines Gabelblattes (67; 68) etwa dem Zwölffachen der Breite B des Zwischenraumes (50) entspricht.

20. Anordnung nach einem der vorhergehenden Ansprüche, **dadurch gekennzeichnet, daß** die Fläche der beiden Gabelblätter (67, 68)und des Zwischenraumes (50) sich im wesentlichen zu einem Rechteck ergänzen, wobei die Breite BG etwa dem Fünffachen der Breite B des Zwischenraumes (50) entspricht.

21. Anordnung nach Anspruch 19 oder 20, **dadurch gekennzeichnet, daß** die Fläche der beiden Gabelblätter (67, 68) etwa 30 cm² beträgt.

## Claims

1. An arrangement for locally immobilising a beating heart, comprising a fork-type platform (14) having two fork plates (46, 48) extending substantially parallel and forming a gap (50) in between, wherein the gap (50) has a width corresponding to 1 to 5 times the width of a coronary vessel (62) to be arranged in the gap (50), and wherein at least one opening (60) is provided in each fork plate (46, 48) adjacent to the gap (50), and furthermore wherein means (54) for looping around the coronary vessel (62) are provided which are insertable through the at least two openings (60) and are fixable to at least one fixing device (52) provided on the fork plates (46, 48).

2. An arrangement according to claim 1, **characterised in that** at least one circular enlargement (66) of the gap (50) is provided.

3. An arrangement according to claim 2, **characterised in that** the circular enlargement (66) has a diameter of 8 mm to 12 mm, in particular 10 mm.

4. An arrangement according to any one of the preceding claims, **characterised in that** the fixing means (52) is at least one profiled stud with a mushroom-shaped cross-section.

5. An arrangement according to any one of the preceding claims, **characterised in that** at least two openings (60) are formed in each fork plate (46, 48).

6. An arrangement according to any one of the preceding claims, **characterised in that** the at least two openings (60) are bores in the fork plates (46, 48).

7. An arrangement according to any one of the preceding claims, **characterised in that** slots (64) are provided in the fork plates (46, 48) and each extend from the gap (50) to an opening (60).

8. An arrangement according to claim 7, **characterised in that** the slots (64) are each hook-shaped or S-shaped.

9. An arrangement according to any one of the preceding claims, **characterised in that** at least one fork plate (46, 48) is chamfered on its underside in the direction of the gap (50).

10. An arrangement according to claim 9, **characterised in that** the chamfer angle is 5 to 15 degrees, in particular 10 degrees.

11. An arrangement according to any one of the preceding claims, **characterised in that** the gap (50) has a width of 4 mm to 10 mm, in particular 6.5 mm.

12. An arrangement according to any one of the preceding claims, **characterised in that** the means (54) for looping around the coronary vessel (62) is at least one thread or at least one vessel loop.

13. An arrangement according to any one of the preceding claims, **characterised in that** the gap (50) has a width corresponding to 1 to 2 times, 1 to 3 times or 2 to 3 times the width of the coronary vessel (62) to be arranged in the space (50).

14. An arrangement according to any one of the preceding claims, **characterised in that** the gap (50) has a width such that a coronary vessel (62) is arrangeable therein so as to be laterally spaced from the fork plates (46, 48), the spaces each being equal to or smaller than the width of the coronary vessel.

15. An arrangement according to any one of the preceding claims, **characterised in that** the fork plates (46, 48) are connected to one another by a bridge (44) having a substantially semi-circular recess between two arms, on each of which is arranged a fork plate (46, 48).

16. An arrangement according to claim 15, **characterised in that** the semi-circular recess has a radius of 2 mm to 5 mm, in particular 3.25 mm.

17. An arrangement according to any one of the preceding claims, **characterised in that** the surfaces of the two fork plates (65, 66) and the gap (50) substantially complement one another to form a circular ring, the outer edge of the segments of the circle being at a distance from a hypothetical centre M of the circle or having a radius R which corresponds to approximately three times the width B of the gap (50).

18. An arrangement according to claim 17, **characterised in that** the area of the two fork plates (65, 66) is approximately 7 cm².

19. An arrangement according to any one claims 1 to 16, **characterised in that** the surfaces of the two fork plates (67, 68) and the gap (50) substantially complement one another to form a rectangle, the length L of the surface of one fork plate (67; 68) corresponding to approximately twelve times the width B of the gap (50).

20. An arrangement according to any one of the preceding claims, **characterised in that** surfaces of the two fork plates (67, 68) and the gap (50) substantially complement one another to form a rectangle, the width BG corresponding to approximately five times the width B of the gap (50).

21. An arrangement according to claim 19 or 20, **characterised in that** the area of the two fork plates (67, 68) is approximately 30 cm².

## Revendications

1. Dispositif pour l'immobilisation locale d'un coeur en train de battre comportant une plate-forme de type fourche (14) avec deux plaques de fourche (46, 48) qui s'étendent sensiblement parallèlement, qui forment entre elles un interstice (50), où l'interstice (50) présente une largeur qui correspond à 1 à 5 fois la largeur d'un vaisseau coronaire (62) qui doit être disposé dans l'interstice (50), et où il est prévu sur les plaques de fourche (46, 48), au voisinage de l'interstice (50), dans chaque cas au moins une ouverture (60), où il est prévu en outre des moyens (54) pour enlacer le vaisseau coronaire (62), qui peuvent être amenés à traverser les ouvertures (60) au nombre d'au moins deux et qui peuvent être fixés à au moins un dispositif de fixation (52) prévu sur les plaques de fourche (46, 48).

2. Dispositif selon la revendication 1 **caractérisé en ce qu'**il est prévu au moins un élargissement circulaire (66) de l'interstice (50).

3. Dispositif selon la revendication 2 **caractérisé en ce que** l'élargissement circulaire (66) a un diamètre de 8 mm à 12 mm, en particulier de 10 mm.

4. Dispositif selon l'une des revendications précédentes **caractérisé en ce que** le moyen de fixation (52) est au moins un bouton profilé à section droite de type champignon.

5. Dispositif selon l'une des revendications précédentes **caractérisé en ce qu'**il est formé dans chaque cas au moins deux ouvertures (60) sur une plaque de fourche (46, 48).

6. Dispositif selon l'une des revendications précédentes **caractérisé en ce que** les ouvertures (60) au nombre d'au moins deux sont des alésages dans les plaques de fourche (46, 48).

7. Dispositif selon l'une des revendications précédentes **caractérisé en ce qu'**il est prévu dans les plaques de fourche (46, 48) des fentes (64) qui s'étendent chacune de l'interstice (50) à une ouverture (60).

8. Dispositif selon la revendication 7 **caractérisé en ce que** les fentes (64) sont chacune en forme de crochet ou de S.

9. Dispositif selon l'une des revendications précédentes **caractérisé en ce qu'**au moins une plaque de fourche (46, 48) est biseautée sur son côté inférieur en direction de l'interstice (50).

10. Dispositif selon la revendication 9 **caractérisé en ce que** l'angle de biseau est de 5 à 15°, en particulier de 10°.

11. Dispositif selon l'une des revendications précédentes **caractérisé en ce que** l'interstice (50) présente une largeur de 4 mm à 10 mm, en particulier de 6,5 mm.

12. Dispositif selon l'une des revendications précédentes **caractérisé en ce que** le moyen (54) pour enlacer le vaisseau coronaire (62) est au moins un fil ou au moins une boucle de vaisseau.

13. Dispositif selon l'une des revendications précédentes **caractérisé en ce que** l'interstice (50) présente une largeur qui correspond à une à deux fois, une à trois fois ou deux à trois fois la largeur du vaisseau coronaire (62) qui doit être disposé dans l'interstice (50).

14. Dispositif selon l'une des revendications précédentes **caractérisé en ce que** l'interstice (50) présente une largeur telle qu'il est possible de disposer dans celui-ci un vaisseau coronaire (62) de telle manière que celui-ci présente des distances latérales aux plaques de fourche (46, 48) qui sont chacune égales ou inférieures à la largeur du vaisseau coronaire.

15. Dispositif selon l'une des revendications précédentes **caractérisé en ce que** les plaques de fourche (46, 48) sont reliées entre elles par un pont (44) qui comporte un évidement sensiblement en forme de demi-cercle entre deux branches sur chacune desquelles est disposée une plaque de fourche (46, 48).

16. Dispositif selon la revendication 15 **caractérisé en ce que** l'évidement en forme de demi-cercle présente un rayon de 2 mm à 5 mm, en particulier de 3,25 mm.

17. Dispositif selon l'une des revendications précédentes **caractérisé en ce que** les surfaces des deux plaques de fourche (65, 66) et de l'interstice (50) se complètent sensiblement en un anneau de cercle, où le bord externe des segments de cercle présente une distance ou un rayon R à un centre fictif M qui correspond sensiblement au triple de la largeur B de l'interstice (50).

18. Dispositif selon la revendication 17 **caractérisé en ce que** la surface des deux plaques de fourche (65, 66) est d'environ 7 cm².

19. Dispositif selon l'une des revendications 1 à 16 **caractérisé en ce que** les surfaces des deux plaques de fourche (67, 68) et de l'interstice (50) se complètent sensiblement en un rectangle, où la longueur L de la surface d'une plaque de fourche (67, 68) correspond sensiblement à douze fois la largeur B de l'interstice (50).

20. Dispositif selon l'une des revendications précédentes **caractérisé en ce que** les surfaces des deux plaques de fourche (67, 68) et de l'interstice (50) se complètent sensiblement en un rectangle, où la largeur BG correspond sensiblement à cinq fois la largeur B de l'interstice (50).

21. Dispositif selon la revendication 19 ou 20 **caractérisé en ce que** la surface des deux plaques de fourche (67, 68) est d'environ 30 cm².
